# EUROPÄISCHE PATENTSCHRIFT

(11) **EP 1 086 949 B1**
(45) Veröffentlichungstag und Bekanntmachung des Hinweises auf die Patenterteilung: **19.02.2003**
(21) Anmeldenummer: 00119865.4
(22) Anmeldetag: 13.09.2000
(51) Int. Cl.: C07D 311/76

(54) **Verfahren zur Herstellung von Isochroman-3-onen**
Process for the preparation of isochroman-3-ones
Procédé de préparation d'isochromane-3-ones

(30) Priorität: 23.09.1999 DE 19945561
(43) Veröffentlichungstag der Anmeldung: 28.03.2001
(73) Patentinhaber: Clariant GmbH, 65929 Frankfurt am Main (DE)
(72) Erfinder: Geissler, Holger, Dr., 55116 Mainz (DE); Pfirmann, Ralf, Dr., 64347 Griesheim (DE)

(56) Entgegenhaltungen:
- EP-A- 0 834 497
- WO-A-00/17186
- CHEMICAL ABSTRACTS, vol. 133, no. 2, 2000 Columbus, Ohio, US; abstract no. 17382p, Seite 638; XP002160855 & JP 2000 159759 A (SAGAMI) 13. Juni 2000 (2000-06-13)

## Beschreibung

Die vorliegende Erfindung betrifft ein vorteilhaftes Verfahren zur Herstellung von Isochroman-3-onen.

Isochroman-3-on ist bei der Synthese von Pharmazeutika und Pflanzenschutzmitteln als Zwischenprodukt von großem Interesse.

Aus der WO 97/12864 geht beispielsweise die Verwendung von Isochroman-3-on als Zwischenprodukt bei der Herstellung von Fungiziden und Pestiziden hervor.

Die Qualität von traditionellen chemischen Prozessen ist in der Regel durch die Raum/Zeitausbeute gegeben. In katalytisch chemischen Prozessen wird dagegen in der Regel die katalytische Wechselzahl (sogenannte "turnover number" oder "TON", d.h. der Wert, der angibt, wie oft ein Katalysatorteilchen in der Reaktion benutzt wird) und die katalytische Wechselfrequenz (sogenannte "turnover frequence" oder "TOF", d.h. der Wert, der angibt, wie oft ein Katalysatorteilchen in einer Stunde in der Reaktion benutzt wird) als Qualitätsmerkmal herangezogen. Die TON und TOF geben im Vergleich mit der Raum/Zeitausbeute zusätzlich Informationen über die Qualität des in der Reaktion eingesetzten Katalysators.

In der Literatur sind verschiedene Verfahren zur Herstellung von Isochroman-3-on bekannt.
So beschreibt Yamamoto in Tetrahedron Lett. 1997, Vol. 38, 3747 bis 3750 eine Synthese von Isochroman-3-on durch Umsetzung von 1,2-Bis-hydroxymethyl-benzol und Kohlenmonoxid in Gegenwart von 1 mol-% eines Palladiumkatalysators und 10 mol-% Jodwasserstoff. Man erhält bei 90°C und 9 MPa Kohlenmonoxid-Druck in Aceton-Wasser als Lösungsmittel nach 42 Stunden Reaktionszeit Isochroman-3-on in isolierter Form mit 56 % Ausbeute.

Als Nachteile dieses Verfahrens sind die Anwesenheit des sehr korrosiv wirkenden Jodwasserstoffs und die recht lange Reaktionszeit zu erwähnen.

Stille beschreibt in J. Am. Chem. Soc. 1980, Vol. 102, 4193 bis 4198 die Synthese von Isochroman-3-on durch Umsetzung von ortho-Brommethylbenzylalkohol, Kohlenmonoxid und Kaliumcarbonat in Gegenwart von 1,6 mol-% eines Palladiumkatalysators und einem Tropfen Hydrazin in Tetrahydrofuran als Lösungsmittel. Man erhält nach 24 Stunden bei 25°C und 0,1 MPa Kohlenmonoxid-Druck Isochroman-3-on in isolierter Form mit einer Ausbeute von 71 %.

Von Nachteil ist, daß der als Ausgangsstoff benötigte ortho-Brommethylbenzylalkohol nicht einfach zugänglich ist. Zudem erschwert die Verwendung von Kaliumcarbonat eine einfache Verfahrensdurchführung (Freisetzen von CO₂). Femer ist eine vergleichsweise lange Reaktionszeit in Kauf zu nehmen.

Aus der WO 97/00850 A1 geht ein zweistufiges Verfahren zur Herstellung von Isochroman-3-on-Derivaten hervor, wobei zunächst ein 1,2-Bis-halomethylbenzolderivat der allgemeinen Formel (A) 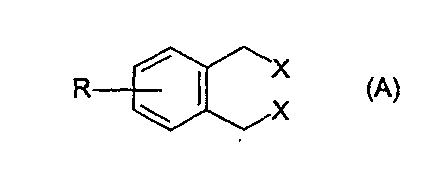 worin R für H, ein Halogen, einen C₁-C₆-Alkyl- oder C₁-C₆-Alkoxy-Rest und X für ein Halogen steht, Kohlenmonoxid und Wasser in Gegenwart eines Wasserstoffhalogenidaufnahmemittels und eines Katalysators in einem organischen Lösungsmittel umgesetzt werden und das als Zwischenprodukt auftretende Salz der ortho-Hydroxymethylphenylessigsäure der allgemeinen Formel (B), worin M für ein Alkali- oder Erdalkalimetall und n für 1 oder 2 steht, 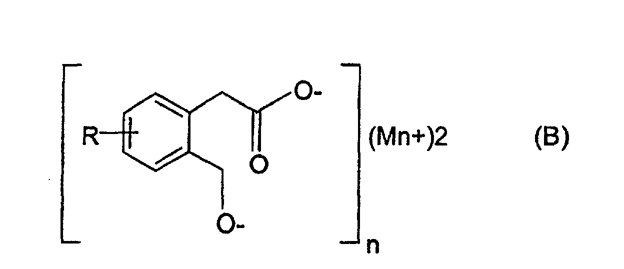 anschließend mit einer Säure behandelt und in das entsprechende Isochroman-3-on umgewandelt wird. Als Katalysator kommen Palladium-, Kobalt- und Eisenkatalysatoren in Betracht. Als Wasserstoffhalogenidaufnahmemittel kommen Basen, insbesondere anorganische Basen, beispielsweise Calciumhydroxid zum Einsatz. In der zweiten Reaktionsstufe dieses Verfahrens wird als Säure beispielsweise Salzsäure verwendet, um die Umwandlung des Salzes des ortho-Hydroxymethylphenylessigsäurederivates der Formel (B) in das entsprechende Isochroman-3-on herbeizuführen. Die maximale TOF beträgt 153 x h⁻¹; TON = 153; Ausbeute 76,7 % (vgl. Ausführungsbeispiel 4). Die maximale TON beträgt 170 (TOF = 24 x h⁻¹); Ausbeute 84,7 % (vgl. Ausführungsbeispiel 17).

Nach diesem Verfahren kann zwar eine Ausbeute von bis zu 87,4 % an Isochroman-3-on erzielt werden, wobei jedoch eine vergleichsweise geringe Menge von 8,75 g α,α'-ortho-Xylylendichlorid (1,2-Bis-chlormethylbenzol) in nicht weniger als 100 g tert.-Butanol umgesetzt wird. Zur weiteren Aufarbeitung wird das Reaktionsgemisch mit Wasser versetzt, unlösliche Feststoffe werden durch Filtration abgetrennt und es wird mehrfach mit Ether extrahiert. Nach Ansäuern mit konzentrierter Salzsäure wird erneut mit Ether extrahiert und aus den gesammelten Etherfraktionen Isochroman-3-on gewonnen (TON = 87; TOF = 4,2 x h⁻¹; vgl auch Ausführungsbeispiel 5).

Bedingt durch Einsatz von Basen im ersten Schritt des Verfahrens und durch das Ansäuern im zweiten Schritt entstehen nicht weniger als 3 Äquivalente einwertiges Salz pro Äquivalent Isochroman-3-on. Nachteilig in diesem Verfahren ist einerseits der Einsatz großer Mengen Lösungsmittel und die Bildung hoher Salzmengen und andererseits die Zweistufigkeit des Verfahrens und die zahlreichen Reinigungs- und Extraktionsschritte sowie die wiederholte Verwendung von Ether als Extraktionsmittel.

Im Hinblick auf die Nachteile der vorstehend geschilderten Verfahren liegt der vorliegenden Erfindung die Aufgabe zugrunde, ein neues Verfahren zur Herstellung von Isochroman-3-onen bereitzustellen, das sich zum einen mit vergleichsweise geringem Aufwand ausführen läßt, zum anderen die zuvor beschriebenen Nachteile der Verfahren des Standes der Technik vermeidet und das gewünschte Produkt in guter Ausbeute und hoher Reinheit zugänglich macht.

Diese Aufgabe wird gelöst durch ein Verfahren zur Herstellung eines Isochroman-3-ons der Formel (I) 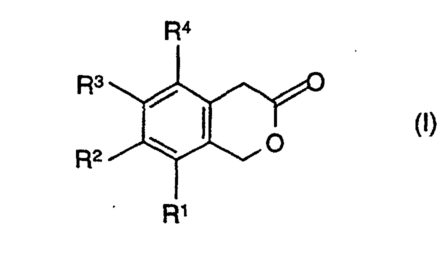 durch Umsetzung eines 1,2-Bis-halogenmethylbenzols der Formel (II) 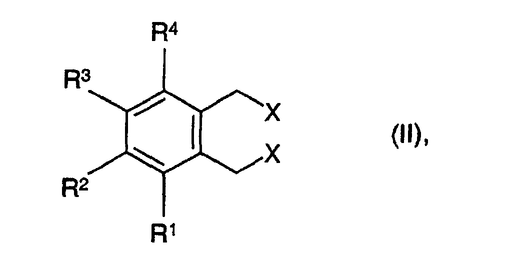 worin X Chlor, Brom oder Jod ist, mit Kohlenmonoxid und einer Verbindung der Formel (III)

R⁵R⁶R⁷C-OH (III)

bei einem CO-Druck von 0,1 bis 50 MPa und einer Temperatur von 20 bis 200°C in Anwesenheit oder Abwesenheit eines ionischen Halogenids, in Gegenwart eines Palladiumkatalysators und eines dipolar aprotischen Lösungsmittels, mit Zusatz von Wasser oder ohne Zusatz von Wasser, wobei in den Formeln (I) und (II) die Reste R¹, R², R³ und R⁴ unabhängig voneinander darstellen:
ein Wasserstoff- oder Fluoratom;
eine NC- oder F₃C-Gruppe;
einen Alkyl-, Alkoxy- oder Acyloxyrest, mit jeweils 1 bis 18 Kohlenstoffatomen; oder
einen C₆-C₁₈-Aryloxy-, Aryl- oder Heteroarylrest, wobei als Heteroatome 1 bis 3 Atome aus der Gruppe O, N und/oder S vorhanden sind;
oder worin mindestens zwei der Reste R¹, R², R³ und R⁴ untereinander verknüpft
sind und mindestens einen aliphatischen oder aromatischen Ring mit 5 bis 18 C-Atomen bilden, und in Formel (III) die Reste R⁵, R⁶ und R⁷ gleich oder verschieden sind und für einen C₁-C₁₈-Alkyl-, einen HOC(=O)-, H₃CC(=O)CH₂- oder (C₆-C₁₈-Aryl)-CH₂-Rest stehen oder mindestens zwei der Reste R⁵, R⁶ und R⁷ untereinander verknüpft sind und mindestens einen aliphatischen oder aromatischen Ring mit 5 bis 18 C-Atomen bilden.

Die Umsetzung des 1,2-Bis-halogenmethylbenzols der Formel (II) läßt sich - belegt anhand eines 1,2-Bis-chlormethylbenzols als Beispiel für eine Verbindung der Formel (II) und anhand von tert.-Butanol als Beispiel für eine Verbindung der Formel (III) - in vereinfachter Form durch die nachfolgende Gleichung schematisch beschreiben.

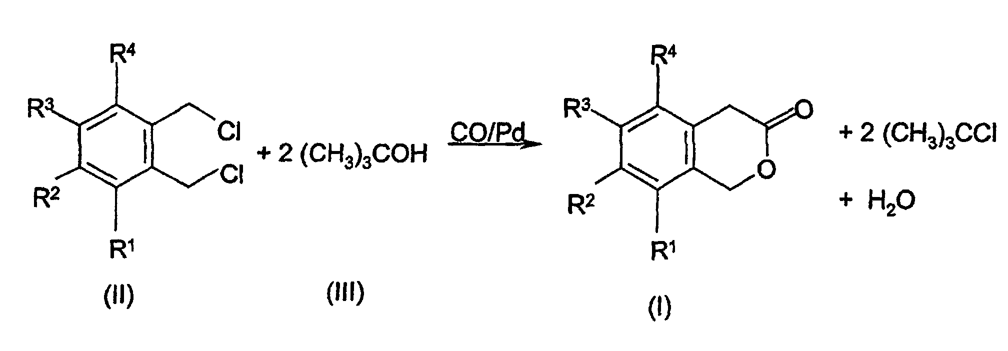

Wie aus dieser hier als Anschauungsbeispiel dienenden Gleichung hervorgeht, entsteht das entsprechende Isochroman-3-on der Formel (I), tert.-Butylchlorid und Wasser.
Das erfindungsgemäße Verfahren ermöglicht es, das 1,2-Bis-halogenmethylbenzol der Formel (II) in Konzentrationen, die signifikant höher als im Verfahren gemäß der WO 97/00850 A1 liegen, umzusetzen. Dadurch wird vorteilhafterweise die Raum/Zeitausbeute erhöht und eine technische Durchführung in entsprechendem Maße begünstigt.

Ein weiterer Vorteil besteht darin, daß im Vergleich zum Verfahren der WO 97/00850 A1 nicht das Salz der Formel (B) zu bilden ist und auch nicht das Salz anfällt, das durch die Reaktion des Wasserstoffhalogenidaufnahmemittel (Base) mit Halogenwasserstoff entsteht. Somit läuft das erfindungsgemäße Verfahren in Abwesenheit eines derartigen Wasserstoffhalogenidaufnahmemittels ab und es kann zudem vorteilhafterweise auf den zweiten Reaktionsschritt unter Zusatz von Säure verzichtet werden.

Die Reste R¹, R², R³ und R⁴ bedeuten insbesondere unabhängig voneinander Wasserstoff, Fluor, C₁-C₄-Alkyl oder C₁-C₄-Alkoxy oder zwei der Reste R¹, R², R³ und R⁴ sind untereinander verknüpft und bilden einen aliphatischen oder aromatischen Ring mit 5 bis 10 C-Atomen. Vorzugsweise stehen R¹, R², R³ und R⁴ unabhängig voneinander für Wasserstoff, Fluor oder C₁-C₄-Alkyl oder C₁-C₄-Alkoxy, insbesondere für Wasserstoff, Fluor oder C₁-C₄-Alkyl.

In den Formeln (I) und (II) können zwei, drei oder vier, insbesondere drei oder vier der Reste R¹, R², R³ und R⁴ Wasserstoff bedeuten.

Man kann in das Verfahren mit gutem Erfolg ein 1,2-Bis-halogenmethylbenzol der Formel (II), worin X für Chlor oder Brom, insbesondere für Chlor steht, einsetzen.

Man setzt mit gutem Erfolg eine Verbindung der Formel (III) ein, worin die Reste R⁵, R⁶ und R⁷ gleich oder verschieden sind und für einen C₁-C₁₈-Alkyl- oder (C₆-C₁₈-Aryl)-CH₂-Rest, insbesondere für einen C₁-C₁₂-Alkylrest, bevorzugt für einen C₁-C₈-Alkyl-rest stehen. Der Alkylrest kann geradkettig oder verzweigt sein und ist insbesondere geradkettig.

Von besonderem Interesse sind Verbindungen der Formel (III) worin einer der Reste R⁵, R⁶ und R⁷ für einen C₁-C₁₂-Alkylrest, insbesondere für einen C₁-C₈-Alkylrest und die verbleibenden Reste für einen Ethyl- oder Methylrest, insbesondere für einen Methylrest stehen.

Besonders einfach gestaltet sich das erfindungsgemäße Verfahren, indem man tert.-Butanol als Verbindung der Formel III einsetzt.

Man setzt die Verbindung der Formel (III) entsprechend einer Menge von 0,8 bis 10, insbesondere 0,9 bis 3, bevorzugt 1 bis 2,5 Mol je Mol 1,2-Bishalogenmethylbenzol ein.

Wie eingangs erwähnt, kann man das Verfahren in Anwesenheit oder Abwesenheit eines ionischen Halogenids durchführen.

Üblicherweise ist das ionische Halogenid ein Alkali-, Ammonium- oder Phosphoniumhalogenid, insbesondere ein Alkali- oder Ammoniumhalogenid, wobei Halogenid die Bedeutung Chlorid, Bromid oder Jodid, insbesondere Chlorid oder Bromid, bevorzugt Chlorid hat.

Man kann als ionisches Halogenid Ammoniumbromid, Lithiumbromid, Natriumbromid, Kaliumbromid, Tetrabutylphosphoniumbromid, Ammoniumchlorid, Dimethylammoniumchlorid, Diethanolammoniumchlorid, Lithiumchlorid, Natriumchlorid, Kaliumchlorid, Tetrabutylphosphoniumchlorid, Ammoniumjodid, Lithiumjodid, Natriumjodid, Kaliumjodid und/oder Tetrabutylphosphoniumjodid, insbesondere Lithiumchlorid, Ammoniumchlorid, Dimethylammoniumchlorid, und/oder Diethanolammoniumchlorid einsetzen.

An dieser Stelle sei darauf hingewiesen, daß man auf die Anwesenheit des ionischen Halogenids verzichten und das Verfahren insbesondere in Abwesenheit des ionischen Halogenids durchführen kann.

Man kann in das Verfahren einen Palladiumkatalysator, der auf einem Trägermaterial aufgebrachtes Palladium enthält, einsetzen. Ein derartiger Palladium-Träger-Katalysator hat den Vorzug, daß er sich aus dem Reaktionsgemisch, beispielsweise durch Filtration, auf einfache Weise abtrennen läßt.

In einer Reihe von Fällen hat es sich bewährt, daß der Palladiumkatalysator mindestens eine Palladium-(II)-Verbindung, insbesondere PdCl₂, PdBr₂ oder Pd(OAc)₂, bevorzugt PdCl₂, oder mindestens eine Palladium-(0)-Verbindung, insbesondere Pd₂dba₃, worin dba für Dibenzylidenaceton steht, Pd(P(C₆H₅)₃)₄ oder Pd(η⁴-C₈H₁₂)₂, bevorzugt Pd₂dba₃ enthält.

In einer Reihe von Fällen hat es sich ferner als günstig erwiesen, daß der Palladiumkatalysator zusätzlich einen Liganden, insbesondere eine Phosphinverbindung, enthält.

Als Phosphinverbindung kommt beispielsweise ein Monophosphin, insbesondere ein Tri-(C₁-C₆-alkyl)phosphin oder ein Triarylphosphin, oder ein Diphosphin in Betracht. Mit gutem Erfolg kann man Triphenylphosphin, Tritolylphosphin, Bis(diphenylphosphino)ethan, 1,3-Bis(diphenylphosphino)propan oder 1,4-Bis(diphenylphosphino)butan, insbesondere Triphenylphosphin einsetzen.

Nach einer besonderen Ausführungsform enthält der Palladiumkatalysator eine Bis(triphenylphosphin)-palladium(II)-Verbindung, beispielsweise Bis(triphenylphosphin)-palladium(II)-chlorid oder Bis(triphenylphosphin)-palladium(II)-bromid.

Man setzt den Palladiumkatalysator üblicherweise entsprechend einer Menge von 0,00001 bis 0,3 Mol Palladium, insbesondere 0,000025 bis 0,2 Mol Palladium, bevorzugt 0,00005 bis 0,1 Mol Palladium je Mol 1,2-Bis-halogenmethylbenzol ein.

In einer Vielzahl von Fällen genügt es, die Umsetzung bei einem CO-Druck von 0,1 bis 20 MPa, insbesondere 0,5 bis 10, bevorzugt 1,0 bis 6 MPa durchzuführen.

Üblicherweise kann man die Umsetzung bei einer Temperatur von 50 bis 170°C, insbesondere 70 bis 160°C, bevorzugt 90 bis 150°C mit gutem Erfolg durchführen.

Wie eingangs bereits erwähnt, führt man die Umsetzung in Gegenwart eines dipolar aprotischen Lösungsmittels durch. Es ist üblicherweise ausreichend, das dipolar aprotische Lösungsmittel in einer Menge von 30 bis 95, insbesondere 50 bis 90, bevorzugt 60 bis 85 Gew.-%, bezogen auf gesamtes Einsatzgemisch, einzusetzen.

Als dipolar aprotisches Lösungsmittel eignet sich Dioxan, Tetrahydrofuran, ein N-(C₁-C₁₈-Alkyl)pyrrolidon, Ethylenglykoldimethylether, ein C₁-C₄-Alkylester einer aliphatischen C₁-C₆-Carbonsäure, ein C₁-C₆-Dialkylether, ein N,N-Di-(C₁-C₄alkyl)amid einer aliphatischen C₁-C₄-Carbonsäure, Sulfolan, ein 1,3-Di-(C₁-C₈-alkyl)-2-imidazolidinon, ein N-(C₁-C₈-Alkyl)caprolactam, ein N,N,N',N'-Tetra-(C₁-C₈alkyl)harnstoff, ein 1,3-Di-(C₁-C₈-alkyl)-3,4,5,6-tetrahydro-2(1H)-pyrimidon, ein N,N,N',N'-Tetra-(C₁-C₈-alkyl)sulfamid, 4-Formylmorpholin, 1-Formylpiperidin oder 1-Formylpyrrolidin, insbesondere ein N-(C₁-C₁₈-Alkyl)pyrrolidon, ein N,N-Di(C₁-C₄-Alkyl)amid einer aliphatischen C₁-C₄-Carbonsäure, 4-Formylmorpholin, 1-Formylpiperidin oder 1-Formylpyrrolidin, bevorzugt N-Methylpyrrolidon, N-Octylpyrrolidon, N-Dodecylpyrrolidon, N,N-Dimethylformamid, N,N-Dimethylacetamid, 4-Formylmorpholin, 1-Formylpiperidin oder 1-Formylpyrrolidin, besonders bevorzugt N-Methylpyrrolidon, N,N-Dimethylformamid oder N,N-Dimethylacetamid, ganz besonders bevorzugt N-Methylpyrrolidon. Es lassen sich auch Mischungen der vorstehend genannten dipolar aprotischen Lösungsmittel verwenden.

Wie eingangs erwähnt, führt man das Verfahren mit Zusatz von Wasser oder ohne Zusatz von Wasser, insbesondere mit Zusatz von Wasser durch. In vielen Fällen hat sich ein Zusatz von Wasser in untergeordneten Mengen, beispielsweise 0,001 bis 0,3 Mol Wasser je Mol 1,2-Bishalogenmethylbenzol der Formel (II) als günstig erwiesen.

Üblicherweise führt man die Umsetzung mit einer Menge Wasser entsprechend 0,005 bis 0,25, insbesondere 0,01 bis 0,1, bevorzugt 0,02 bis 0,08 Mol Wasser je Mol 1,2-Bis-halogenmethylbenzol der Formel (II) durch.

Nach einer besonderen Ausführungsform des erfindungsgemäßen Verfahrens legt man das 1,2-Bis-halogenmethylbenzol der Formel (II), den Palladiumkatalysator, das dipolar aprotische Lösungsmittel und gegebenenfalls das ionische Halogenid vor, stellt den CO-Druck und die Temperatur ein und dosiert ein aus Wasser und dipolar aprotischen Lösungsmittel bestehendes Gemisch und anschließend die Verbindung der Formel (III) oder ein aus der Verbindung der Formel (III) und dipolar aprotischem Lösungsmittel bestehendes Gemisch zu.

Während der Umsetzung sorgt man für eine gute Durchmischung der Reaktionspartner, um einen zügigen Reaktionsverlauf zu gewährleisten.

Das erfindungsgemäße Verfahren eignet sich sowohl für eine kontinuierliche als auch diskontinuierliche Durchführung.

Die Reaktion wird in der Regel bei einem H₀-Wert ≤ 7 durchgeführt, insbesondere bei H₀ = -3 bis 7, bevorzugt bei -2 bis 6. Es ist allerdings auch möglich, die Reaktion bei einem H₀-Wert von -1 bis 5, insbesondere bei -1 bis 4 durchzuführen. Der H₀-Wert, der ein Maß für die Acidität eines Lösungsmittel ist und für den für verdünnte Lösungen H₀ ≈pH gilt, ist im Hollemann-Wiberg "Lehrbuch der Anorganischen Chemie", 91-100. Auflage, Verlag Walter de Gruyter, Berlin 1985, auf den Seiten 246-248 beschrieben. Kislina et al. beschreiben zum Beispiel die Acidität von HCI in N,N-Dimethylformamid in Russ. Chem. Bull., 1994, Vol. 43, auf den Seiten 960-963. In der Regel stellt sich der entsprechende H₀-Wert im Verlauf einer Reaktion von selbst ein, so daß zusätzliche Maßnahmen zur Einstellung des H₀-Wertes meistens nicht erforderlich sind.

Der H₀-Wert geht aus nachfolgender Gleichung hervor:
H₀ = pK_{S,In} + log C_{In}/C_{InH}⁺ (Hammettsche Aciditätsfunktion). Hierbei steht In für die Indikatorbase und InH⁺ für die protonierte Form der Indikatorbase.
Die nachfolgenden Beispiele beschreiben die Erfindung, ohne sie zu beschränken.

### Experimenteller Teil

### Beispiel 1

### Herstellung von Isochroman-3-on

299 g 1,2-Bis-chlormethylbenzol und 75 mg Palladium(II)-chlorid werden in 725 g N-Methyl-pyrrolidon gelöst und in einem 2000 ml Autoklaven aus HC 4 Stahl unter Schutzgasatmosphäre (Argon) vermischt. Anschließend wird Kohlenmonoxid mit einem Druck von 2 MPa hinzugegeben und die Temperatur auf 150°C erhöht. Bei 150°C und 2,5 MPa Druck werden innerhalb von 2 Minuten 1,7 g eines Gemisches aus Wasser/N-Methylpyrrolidon (Gewichtsverhältnis 1:1) und anschließend innerhalb von 165 Minuten 415 g eines Gemisches aus tert-Butanol/N-Methylpyrrolidon (Gewichtsverhältnis 2:1) zudosiert. Der Reaktionsdruck wird während der Zudosierung konstant auf 4 MPa gehalten. Nach dem Zudosieren läßt man 60 Minuten unter Rühren nachreagieren, kühlt ab und entleert den Autoklaven.

Man erhält 1445 g Reaktionsmischung. Ein gaschromatographische Analyse zeigt, daß die Reaktionsmischung 168 g Isochroman-3-on enthält, entsprechend einer Ausbeute von 66,4 % Isochroman-3-on, bezogen auf eingesetztes 1,2-Bis-chlormethylbenzol (TON = 2649; TOF = 706 x h⁻¹).

### Vergleichsbeispiel 1

### Herstellung von Isochroman-3-on ohne Zusatz eines dipolar aprotischen Lösungsmittels

35,0 g Bis-chlormethylbenzol, 35 mg Palladium(II)-chlorid und 0,12 g Wasser werden in 85 ml tert.-Butanol gelöst und in einem 300 ml Autoklav aus HC 256 Stahl unter Schutzgasatmosphäre (Argon) vermischt. Anschließend wird Kohlenmonoxid mit einem Druck von 1,8 MPa hinzugegeben und die Temperatur auf 100°C erhöht. Während der Temperaturerhöhung und bei der Temperatur von 100°C wird keine Kohlenmonoxidaufnahme beobachtet. Anschließend wird die Temperatur langsam auf 130°C erhöht, Kohlenmonoxid wird hierbei auch nicht aufgenommen.
Bei einem Druck von 2,3 MPa wird nochmals 35 mg Palladium(II)-chlorid gelöst in 5 ml tert.-Butanol und 0,12 g Wasser zudosiert. Man beobachtet keine Aufnahme von Kohlenmonoxid. Eine weitere Dosierung von 2 ml Wasser/tert.-Butanol (1:1) führt ebenfalls zu keiner Reaktion.
Nach weiteren 2 Stunden wird der Autoklav abgekühlt und entspannt. Eine gaschromatographische Analyse zeigt, daß die Reaktionsmischung kein 3-Isochromanon enthält.

### Vergleichsbeispiel 2

### Herstellung von Isochroman-3-on unter Zusatz von Calciumhydroxid als Wasserstoffhalogenidaufnahmemittel

52,5 g 1,2-Bis-chlormethylbenzol, 1,05 g Bis(triphenylphosphin)-palladium(II)-chlorid und 0,87 g Triphenylphosphin werden in 100 ml tert.-Butanol vermischt und in einem 500 ml Glasautoklaven unter Schutzgasatmosphäre (Argon) mit 48,0 g Wasser und 46,8 g Calciumhydroxid suspendiert.

Anschließend wird unter Kohlenmonoxidatmosphäre die Temperatur auf 70°C erhöht. Die unter starker Rührung bei 60°C angefangene Kohlenmonoxidaufnahme ist nach 2 Stunden beendet und man erhält einen dickflüssigen Brei. Die Zugabe von weiteren 1,05 g Bis(triphenylphosphin)-palladium(II)-chlorid und 0,87 g Triphenylphosphin führt zu keiner weiteren Kohlenmonoxidaufnahme.

Nach dem Abkühlen wird mit Salzsäure auf pH 1 angesäuert und mit Diethylether extrahiert. Man erhält 383 g Wasserphase und 299 g organische Phase. Die Wasserphase enthält nach gaschromatographischer Analyse kein Isochroman-3-on, und die organische Phase enthält nach gaschromatographischer Analyse 10,5 g Isochroman-3-on (Ausbeute = 24 %; TON = 46; TOF = 15,4 x h⁻¹).

## Patentansprüche

1. Verfahren zur Herstellung eines lsochroman-3-ons der Formel (I) 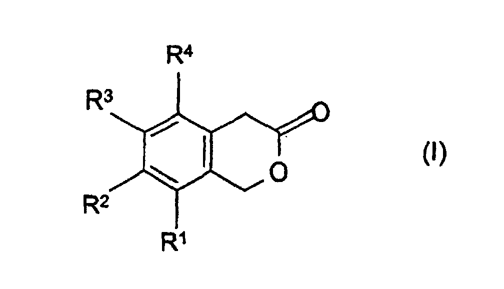 durch Umsetzung eines 1,2-Bis-halogenmethylbenzols der Formel (II) 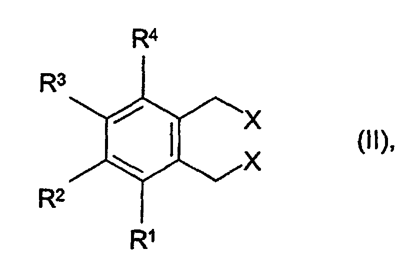 worin X Chlor, Brom oder Jod ist, mit Kohlenmonoxid und einer Verbindung der Formel (III)
R⁵R⁶R⁷C-OH (III)
bei einem CO-Druck von 0,1 bis 50 MPa und einer Temperatur von 20 bis 200°C in Anwesenheit oder Abwesenheit eines ionischen Halogenids, in Gegenwart eines Palladiumkatalysators und eines dipolar aprotischen Lösungsmittels, mit Zusatz von Wasser oder ohne Zusatz von Wasser, wobei in den Formeln (I) und (II) die Reste R¹, R², R³ und R⁴ unabhängig voneinander darstellen:
ein Wasserstoff- oder Fluoratom;
eine NC- oder F₃C-Gruppe;
einen Alkyl-, Alkoxy- oder Acyloxyrest, mit jeweils 1 bis 18 Kohlenstoffatomen; oder einen C₆-C₁₈-Aryloxy-, Aryl- oder Heteroarylrest, wobei als Heteroatome 1 bis 3 Atome aus der Gruppe O, N und/oder S vorhanden sind;
oder worin mindestens zwei der Reste R¹, R², R³ und R⁴ untereinander verknüpft sind und mindestens einen aliphatischen oder aromatischen Ring mit 5 bis 18 C-Atomen bilden, und in Formel (III) die Reste R⁵, R⁶ und R⁷ gleich oder verschieden sind und für einen C₁-C₁₈-Alkyl-, einen HOC(=O)-, H₃CC(=O)CH₂- oder (C₆-C₁₈-Aryl)-CH₂-Rest stehen oder mindestens zwei der Reste R⁵, R⁶ und R⁷ untereinander verknüpft sind und mindestens einen aliphatischen oder aromatischen Ring mit 5 bis 18 C-Atomen bilden.

2. Verfahren nach Anspruch 1, **dadurch gekennzeichnet, daß** die Reste R¹, R², R³ und R⁴ unabhängig voneinander Wasserstoff, Fluor, C₁-C₄-Alkyl oder C₁-C₄-Alkoxy bedeuten oder zwei der Reste R¹, R², R³ und R⁴ untereinander verknüpft sind und einen aliphatischen oder aromatischen Ring mit 5 bis 10 C-Atomen bilden.

3. Verfahren nach Anspruch 1 oder 2, **dadurch gekennzeichnet, daß** zwei, drei oder vier der Reste R¹, R², R³ und R⁴ Wasserstoff bedeuten.

4. Verfahren nach einem oder mehreren der Ansprüche 1 bis 3, **dadurch gekennzeichnet, daß** man eine Verbindung der Formel (III), worin R⁵, R⁶ und R⁷ gleich oder verschieden sind und für einen C₁-C₈-Alkylrest stehen, einsetzt.

5. Verfahren nach einem oder mehreren der Ansprüche 1 bis 4, **dadurch gekennzeichnet, daß** man eine Verbindung der Formel (III), worin einer der Reste R⁵, R⁶ und R⁷ für einen C₁-C₈-Alkylrest und die verbleibenden Reste für einen Methylrest stehen, einsetzt.

6. Verfahren nach einem oder mehreren der Ansprüche 1 bis 5, **dadurch gekennzeichnet, daß** man tert.-Butanol als Verbindung der Formel (III) einsetzt.

7. Verfahren nach einem oder mehreren der Ansprüche 1 bis 6, **dadurch gekennzeichnet, daß** man die Verbindung der Formel (III) entsprechend einer Menge von 0,8 bis 10 Mol, je Mol 1,2-Bis-halogenmethylbenzol der Formel (II) einsetzt.

8. Verfahren nach einem oder mehreren der Ansprüche 1 bis 7, **dadurch gekennzeichnet, daß** das ionische Halogenid ein Alkali-, Ammonium- oder Phosphoniumhalogenid ist, wobei Halogenid die Bedeutung Chlorid, Bromid oder lodid hat.

9. Verfahren nach einem oder mehreren der Ansprüche 1 bis 8, **dadurch gekennzeichnet, daß** der Palladiumkatalysator auf einem Trägermaterial aufgebrachtes metallisches Palladium oder mindestens eine Palladium-(ll)-Verbindung oder mindestens eine Palladium-(0)-Verbindung enthält.

10. Verfahren nach einem oder mehreren der Ansprüche 1 bis 9, **dadurch gekennzeichnet, daß** der Palladiumkatalysator eine Phosphinverbindung zusätzlich als Ligand enthält.

11. Verfahren nach Anspruch 9, **dadurch gekennzeichnet, daß** die Phosphinverbindung Triphenylphosphin, Tritolylphosphin, Bis(diphenylphosphino)ethan, 1,3-Bis(diphenylphosphino)propan oder 1,4-Bis(diphenylphosphino)butan ist.

12. Verfahren nach einem oder mehreren der Ansprüche 1 bis 11, **dadurch gekennzeichnet, daß** der Palladiumkatalysator Bis(triphenylphosphin)-palladium(II)-chlorid oder Bis(triphenylphosphin)palladium(II)-bromid enthält.

13. Verfahren nach einem oder mehreren der Ansprüche 1 bis 12, **dadurch gekennzeichnet, daß** man den Palladiumkatalysator entsprechend einer Menge von 0,00001 bis 0,3 Mol Palladium, je Mol 1,2-Bis-halogenmethylbenzol einsetzt.

14. Verfahren nach einem oder mehreren der Ansprüche 1 bis 13, **dadurch gekennzeichnet, daß** das dipolar aprotische Lösungsmittel Dioxan, Tetrahydrofuran, ein N-(C₁-C₁₈-Alkyl)pyrrolidon, Ethylenglykoldimethylether, ein C₁-C₄-Alkylester einer aliphatischen C₁-C₆-Carbonsäure, ein C₁-C₆-Dialkylether, ein N,N-Di-(C₁-C₄alkyl)amid einer aliphatischen C₁-C₄-Carbonsäure, Sulfolan, 1,3-Di-(C₁-C₈-alkyl)-2-imidazolidinon, ein N-(C₁-C₈-Alkyl)caprolactam, ein N,N,N',N'-Tetra-(C₁-C₈-alkyl)-harnstoff, ein 1,3-Di-(C₁-C₈-alkyl)-3,4,5,6-tetrahydro-2(1H)-pyrimidon, ein N,N,N',N'-Tetra-(C₁-C₈-alkyl)sulfamid, 4-Formylmorpholin, 1-Formylpiperidin oder 1-Formylpyrrolidin ist.

15. Verfahren nach einem oder mehreren der Ansprüche 1 bis 14, **dadurch gekennzeichnet, daß** das dipolar aprotische Lösungsmittel N-Methylpyrrolidon, N-Octylpyrrolidon, N-Dodecylpyrrolidon, N,N-Dimethylformamid, N,N-Dimethylacetamid, 4-Formylmorpholin, 1-Formylpiperidin oder 1-Formylpyrrolidin ist.

16. Verfahren nach einem der Ansprüche 1 bis 15, **dadurch gekennzeichnet, daß** man Wasser entsprechend einer Menge von 0,005 bis 0,25 Mol, je Mol 1,2-Bis-halogenmethylbenzol der Formel (II), einsetzt.

## Claims

1. A process for the preparation of an isochroman-3-one of the formula (I) 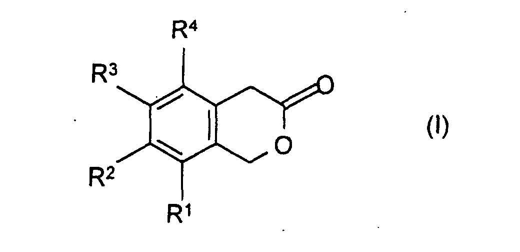 by reaction of a 1,2-bishalomethylbenzene of the formula (II) 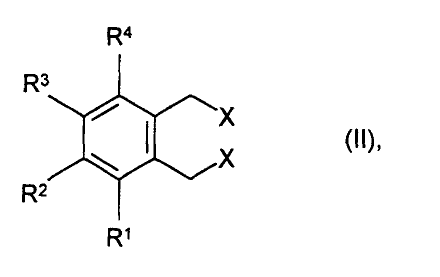 in which X is chlorine, bromine or iodine, with carbon monoxide and a compound of the formula (III)
R⁵R⁶R⁷C-OH (III)
at a CO pressure of 0.1 to 50 MPa and a temperature of 20 to 200°C in the presence or absence of an ionic halide, in the presence of a palladium catalyst and of a dipolar aprotic solvent, with addition of water or without addition of water, where in the formulae (I) and (II) the radicals R¹, R², R³ and R⁴ independently of one another are:
a hydrogen or fluorine atom;
an NC or F₃C group;
an alkyl, alkoxy or acyloxy radical, in each case having 1 to 18 carbon atoms; or a C₆-C₁₈-aryloxy, aryl or heteroaryl radical, where 1 to 3 atoms from the group consisting of O, N and/or S are present as heteroatoms;
or in which at least two of the radicals R¹, R², R³ and R⁴ are linked to one another and form at least one. aliphatic or aromatic ring having 5 to 18 carbon atoms, and in formula (III) the radicals R⁵, R⁶ and R⁷ are identical or different and are a C₁-C₁₈-alkyl, an HOC(=O)-, H₃CC(=O)CH₂- or (C₆-C₁₈-aryl)-CH₂- radical or at least two of the radicals R⁵, R⁶ and R⁷ are linked to one another and form at least one aliphatic or aromatic ring having 5 to 18 carbon atoms.

2. The process as claimed in claim 1, wherein the radicals R¹, R², R³ and R⁴ independently of one another are hydrogen, fluorine, C₁-C₄-alkyl or C₁-C₄-alkoxy or two of the radicals R¹, R², R³ and R⁴ are linked to one another and form an aliphatic or aromatic ring having 5 to 10 carbon atoms.

3. The process as claimed in claim 1 or 2, wherein two, three or four of the radicals R¹, R², R³ and R⁴ are hydrogen.

4. The process as claimed in one or more of claims 1 to 3, wherein a compound of the formula (III) is employed in which R⁵, R⁶ and R⁷ are identical or different and are a C₁-C₈-alkyl radical.

5. The process as claimed in one or more of claims 1 to 4, wherein a compound of the formula (III) is employed in which one of the radicals R⁵, R⁶ and R⁷ is a C₁-C₈-alkyl radical and the remaining radicals are a methyl radical.

6. The process as claimed in one or more of claims 1 to 5, wherein tert-butanol is employed as the compound of the formula (III).

7. The process as claimed in one or more of claims 1 to 6, wherein the compound of the formula (III) is employed corresponding to an amount of 0.8 to 10 mol per mole of 1,2-bishalomethylbenzene of the formula (II).

8. The process as claimed in one or more of claims 1 to 7, wherein the ionic halide is an alkali metal, ammonium or phosphonium halide, where halide has the meaning chloride, bromide or iodide.

9. The process as claimed in one or more of claims 1 to 8, wherein the palladium catalyst contains metallic palladium or at least one palladium(II) compound or at least one palladium(0) compound applied to a support material.

10. The process as claimed in one or more of claims 1 to 9, wherein the palladium catalyst contains a phosphine compound in addition as a ligand.

11. The process as claimed in claim 9, wherein the phosphine compound is triphenylphosphine, tritolylphosphine, bis(diphenylphosphino)ethane, 1,3-bis(diphenylphosphino)propane or 1,4-bis(diphenylphosphino)butane.

12. The process as claimed in one or more of claims 1 to 11, wherein the palladium catalyst is bis(triphenylphosphine)palladium(II) chloride or bis(triphenylphosphine)palladium(II) bromide.

13. The process as claimed in one or more of claims 1 to 12, wherein the palladium catalyst is employed corresponding to an amount of 0.00001 to 0.3 mol of palladium per mole of 1,2-bishalomethylbenzene.

14. The process as claimed in one or more of claims 1 to 13, wherein the dipolar aprotic solvent is dioxane, tetrahydrofuran, an N-(C₁-C₁₈-alkyl)pyrrolidone, ethylene glycol dimethyl ether, a C₁-C₄-alkyl ester of an aliphatic C₁-C₆-carboxylic acid, a C₁-C₆-dialkyl ether, an N,N-di-(C₁-C₄-alkyl)amide of an aliphatic C₁-C₄-carboxylic acid, sulfolane, a 1,3-di-(C₁-C₈-alkyl)-2-imidazolidinone, an N-(C₁-C₈-alkyl)caprolactam, an N,N,N',N'-tetra-(C₁-C₈-alkyl)urea, a 1,3-di-(C₁-C₈-alkyl)-3,4,5,6-tetrahydro-2(1H)-pyrimidone, an N,N,N',N'-tetra-(C₁-C₈-alkyl)sulfamide, 4-formylmorpholine, 1-formylpiperidine or 1-formylpyrrolidine.

15. The process as claimed in one or more of claims 1 to 14, wherein the dipolar aprotic solvent is N-methylpyrrolidone, N-octylpyrrolidone, N-dodecylpyrrolidone, N,N-dimethylformamide, N,N-dimethylacetamide, 4-formylmorpholine, 1-formylpiperidine or 1-formylpyrrolidine.

16. The process as claimed in one of claims 1 to 15, wherein water is employed corresponding to an amount of 0.005 to 0.25 mol per mole of 1,2-bishalomethylbenzene of the formula (II).

## Revendications

1. Procédé pour la préparation d'une isochroman-3-one de formule (I) 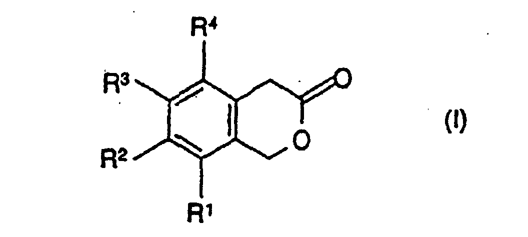 par réaction d'un 1,2-bis-halogénométhylbenzène de formule (II) 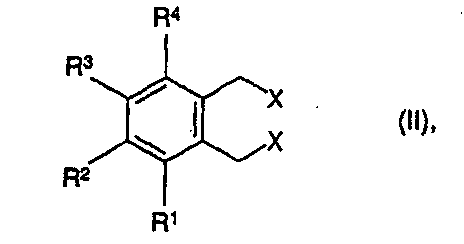 où X représente un atome de chlore, de brome ou d'iode, avec le monoxyde de carbone et un composé de formule (III)
R⁵R⁶R⁷C-OH (III)
sous une pression de CO de 0,1 à 50 MPa et à une température de 20 à 200°C en présence ou en l'absence d'un halogénure ionique, en présence d'un catalyseur au palladium et d'un solvant dipolaire aprotique, avec l'ajout d'eau ou sans ajout d'eau, les restes R¹, R², R³ et R⁴ dans les formules (I) et (II) représentent, indépendamment les uns des autres :
un atome d'hydrogène ou un atome de fluor ;
un groupe NC ou F₃C ;
un reste alkyle, alkoxy ou acyloxy, chacun ayant de 1 à 18 atomes de carbone ; ou un reste (aryl en C₆-C₁₈)-oxy, aryle ou hétéroaryle, 1 à 3 atomes pris dans le groupe comprenant des atomes de O, N et/ou S étant présents en tant qu'hétéroatomes ;
ou dans laquelle au moins deux des restes R¹, R², R³ et R⁴ sont reliés entre eux et forment au moins un cycle aliphatique ou aromatique présentant 5 à 18 atomes de carbone, et à la formule (III), les restes R⁵, R⁶ et R⁷ sont identiques ou différents et représentent un reste alkyle en C₁-C₁₈, HOC(=O)-, H₃CC(=O)CH₂- ou (aryl en C₆-C₁₈)-CH₂- ou au moins deux des restes R⁵, R⁶ et R⁷ sont reliés les uns aux autres et forment au moins un cycle aliphatique ou aromatique présentant 5 à 18 atomes de carbone.

2. Procédé selon la revendication 1, **caractérisé en ce que** les restes R¹, R², R³ et R⁴ représentent, indépendamment les uns des autres, un atome d'hydrogène, un atome de fluor, un groupe alkyle en C₁-C₄ ou alkoxy en C₁-C₄, ou deux restés R¹, R², R³ et R⁴ sont reliés les uns aux autres et forment un cycle aliphatique ou aromatique de 5 à 10 atomes de carbone.

3. Procédé selon la revendication 1 ou 2, **caractérisé en ce que** deux, trois ou quatre des restes R¹, R², R³ et R⁴ représentent des atomes d'hydrogène.

4. Procédé selon une ou plusieurs des revendications 1 à 3, **caractérisé en ce qu'**on utilise un composé de formule (III), où R⁵, R⁶ et R⁷ sont identiques ou différents et représentent un reste alkyle en C₁-C₈.

5. Procédé selon une ou plusieurs des revendications 1 à 4, **caractérisé en ce qu'**on utilise un composé de formule (III), où un des restes R⁵, R⁶ et R⁷ représente un reste alkyle en C₁-C₈ et les autres restes représentent un reste méthyle.

6. Procédé selon une ou plusieurs des revendications 1 à 5, **caractérisé en ce qu'**on utilise du tert.-butanol en tant que composé de formule (III).

7. Procédé selon une ou plusieurs des revendications 1 à 6, **caractérisé en ce qu'**on utilise le composé de formule (III) dans une quantité de 0,8 à 10 moles de 1,2-bis-halogénométhylbenzène de formule (II).

8. Procédé selon une ou plusieurs des revendications 1 à 7, **caractérisé en ce que** l'halogénure ionique est un halogénure alcalin, d'ammonium ou de phosphonium, halogénure signifie chlorure, bromure ou iodure.

9. Procédé selon une ou plusieurs des revendications 1 à 8, **caractérisé en ce que** le catalyseur au palladium est le palladium métal déposé sur un support ou renferme au moins un composé de palladium(II) ou au moins un composé de palladium(0).

10. Procédé selon une ou plusieurs des revendications 1 à 9, **caractérisé en ce que** le catalyseur au palladium renferme de plus un composé de phosphine en tant que ligand.

11. Procédé selon la revendication 9, **caractérisé en ce que** le composé de phosphine est la triphénylphosphine, la tritolylphosphine, le bis(diphénylphosphino)-éthane, le 1,3-bis(diphénylphosphino)-propane ou le 1,4-bis(diphénylphosphino)-butane.

12. Procédé selon une ou plusieurs des revendications 1 à 11, **caractérisé en ce que** le catalyseur au palladium renferme le chlorure de bis(triphénylphosphino)palladium(II) ou le bromure de bis(triphénylphosphino)palladium(II).

13. Procédé selon une ou plusieurs des revendications 1 à 12, **caractérisé en ce qu'**on utilise le catalyseur au palladium dans une quantité de 0,00001 à 0,3 mole de palladium par mole de 1,2-bis-halogénométhylbenzène.

14. Procédé selon une ou plusieurs des revendications 1 à 13, **caractérisé en ce qu'**on utilise en tant que solvant aprotique dipolaire le dioxanne, le tétrahydrofuranne, une N-(alkyl en C₁-C₁₈)-pyrrolidone, l'éther diméthylique de l'éthylèneglycol, un ester d'alkyle en C₁-C₄ d'un acide carboxylique en C₁-C₆ aliphatique, un éther dialkylique en C₁-C₆, un N,N-di(alkyl en C₁-C₄)amide d'un acide carboxylique en C₁-C₄ aliphatique, la sulfolane, une 1,3-di(alkyl en C₁-C₈)-2-imidazolinone, un N-(alkyl en C₁-C₈)-caprolactame, une N,N,N',N'-tétra-(alkyl en C₁-C₈)urée, une 1,3-di(alkyl en C₁-C₈)-3,4,5,6-tétrahydro-2(1H)-pyrimidone, un N,N,N',N'-tétra-(alkyl en C₁-C₈)-sulfamide, la 4-formylmorpholine, la 1-formyl-pipéridine ou la 1-formyl-pyrrolidine.

15. Procédé selon une ou plusieurs des revendications 1 à 14, **caractérisé en ce que** le solvant aprotique dipolaire est la N-méthylpyrrolidone, la N-octylpyrrolidone, la N-dodécylpyrrolidone, le N,N-diméthylformamide, le N,N-diméthylacétamide, la 4-formylmorpholine, la 1-formyl-pipéridine ou la 1-formylpyrrolidone.

16. Procédé selon l'une des revendications 1 à 15, **caractérisé en ce qu'**on utilise l'eau dans une quantité de 0,005 à 0,25 mole par mole de 1,2-bis-halogénométhylbenzène de formule (II).
